# EUROPÄISCHE PATENTANMELDUNG

(11) **EP 1 314 407 A1**
(43) Veröffentlichungstag der Anmeldung: **28.05.2003**
(21) Anmeldenummer: 01811120.3
(22) Anmeldetag: 21.11.2001
(51) Int. Cl.: A61F 2/40

(54) **Schultergelenkprothese**

(71) Anmelder: Sulzer Orthopedics Ltd., 6340 Baar (CH)
(72) Erfinder: Rauscher, Markus, 6319 Allenwinden (CH); Wendt, Peter, 8542 Wiesendangen (CH)
(74) Vertreter: Manitz, Finsterwald & Partner Gbr

(57) **Zusammenfassung**

Es wird eine Schultergelenkprothese mit zwei aufeinander gleitenden Lagerkörpern (1, 2; 11, 12), die zum Oberarm (3) mit einem Schaft (5) respektive zum Schulterknochen (4) mit einer Plattform (6) verbindbar sind, gezeigt. Wenn die Verbindung zum Schaft (5) durch einen nicht rotationssymmetrischen konischen Körper (7) mit einem selbsthemmenden Sitz erfolgt, wobei dessen Umfang (8) einen zu einer Längsachse (9) drehfesten und durch die Konizität verkeilten Formschluss mit einer Gegenform (15) im Schaft (5) bildet, damit die Verbindung lösbar und in gleicher Winkellage wiederholt festsetzbar ist, sind grosse Kräfte durch die Verbindung übertragbar.

## Beschreibung

Die Erfindung handelt von einer Schultergelenkprothese mit zwei aufeinander gleitenden Lagerkörpern, die zum Oberarm mit einem Schaft respektive zum Schulterknochen mit einer Plattform verbindbar sind.

Eine derartige Prothese ist in der Patentanmeldung WO 97/25943 gezeigt. Ein mit einem Schaft im Oberarm verbindbarer Lagerkörper besitzt an seiner Unterseite einen vorstehenden Konus mit Kreisquerschnitt und Indexierbohrungen für einen aus dem Schaft vorstehenden Stift, um in verschiedenen Winkelstellungen den Lagerkörper mit seinem Kreiskonus festzusetzen. Bei dieser Anordnung müssen die Indexierbohrungen und der vorstehende Stift ein Mindestspiel zueinander aufweisen, damit Konus und Gegenfläche mit Sicherheit eine Verbindung bilden. Wegen dieses Spiels werden an der Verbindung auch Torsionskräfte übertragen, die nur durch die Haftreibung übertragen werden können und die zusätzlich zu den axialen Belastungen an den Reibstellen auftreten. Ein derartiger Reibschluss ist mit den zusätzlich auftretenden Schubspannungen durch Torsionsbelastung als Verbindung mehr gefährdet, wobei eine Streuung der übertragbaren Kräfte schon aus dem Schlag, mit welchem ein Press-fit erzeugt werden soll, und aus dem Zustand auf den Konusoberflächen entsteht.

Es ist daher Aufgabe der vorliegenden Erfindung eine bessere Verbindung für eine Befestigung am Schaft einer Schultergelenkprothese zu schaffen. Diese Aufgabe wird mit den Kennzeichen des unabhängigen Anspruchs 1 gelöst, indem die Verbindung zum Schaft durch einen nicht rotationssymmetrischen konischen Körper mit einem selbsthemmenden Sitz erfolgt, wobei dessen Umfang einen zu einer Längsachse drehfesten und durch die Konizität verkeilten Formschluss mit einer Gegenform im Schaft bildet, damit die Verbindung lösbar und in gleicher Winkellage wiederholt festsetzbar ist.

Eine solche Anordnung hat den Vorteil, dass bezüglich der Längsachse der Verbindung in beiden Drehrichtungen bereits Anteile von Normalkräften durch die Verspannung im Ruhezustand vorhanden sind, die bei einer Torsionsbelastung entsprechend dem Abstand ihrer Wirkungslinie zur Drehachse entgegen wirken können. Ein weiterer Vorteil besteht darin, dass durch die Abweichung vom Kreisquerschnitt der Konusverbindung bewusst Auflagepunkte gewählt werden können, die an der äusseren konischen Hülse keine nur von Ringspannung und Elastizität des Materials abhängige Aufweitung erfahren, sondern eine zusätzliche Biegebelastung an der Hülse erzeugen, was einer weicheren Federwirkung der Hülse entspricht und damit einen grösseren Aufschiebeweg und mehr Sicherheit für eine erreichte Haltekraft ergibt.

Weitere vorteilhafte Fortbildungen der Erfindung ergeben sich durch die abhängigen Ansprüche 2 bis 11. So ist es vorteilhaft, bei einem elliptischen Umfang des konischen Körpers die angestrebten Berührungspunkte so zu wählen, dass sie in ihrem radialen Abstand zur Längsachse zwischen dem Betrag der grossen und kleinen Halbachse der Ellipse, aber näher bei dem der grossen Halbachse liegen. Ählich ist es bei einem Dreibogengleichdick, bei dem die Berührungspunkte mit ihrem radialen Abstand zur Längsachse zwischen dem Betrag des kleinsten und grössten Bogenabstandes, aber näher bei dem des grössten Bogenabstandes liegen sollten. Gut sind grundsätzlich Berührungspunkte, in denen die Normalkraft mit ihrer Wirkungslinie einen grossen senkrechten Abstand zur Längsachse aufweist.

Die Stärke der konischen Verbindung, die wiederholt lösbar ist, gestattet es auch in Abwandlung zum natürlichen Schultergelenk an einem bereits implantierten Schaft einen konischen Körper mit einer Kugelschale anzubringen, welche auf einem an der Plattform angebrachten Kugelkopf schwenkbar gelagert ist. Diese Umkehr des Gelenks, die weniger Gleiten und hauptsächlich ein Schwenken um den Kugelmittelpunkt zulässt, ist notwendig, wenn die Bänder stark geschädigt sind. Die Stärke der konischen Verbindung gestattet es, auch komplexere Anwendungen, wie sie in der Patentanmeldung FR 94 14962 A1 gezeigt sind, einzubeziehen. In dieser Publikation sind Ausführungsbeispiele mit einem Schaft gezeigt, der als längsgeschlitzte Kugel mit konischer Bohrung endet, um diese über einen konischen Druckkörper in einer kugelförmigen Ausnehmung des Lagerkopfes festzusetzen. Dadurch, dass diese Kugel an den konischen Körper der vorliegenden Erfindung angeformt ist und dieser auch eine Verstellschraube und den konischen Druckkörper aufnimmt, kann zunächst der Schaft implantiert werden, dann die beste Winkellage des Lagerkopfes mit einer als konischer Körper aufgesteckten Manipulierprothese festgelegt werden und unabhängig vom bereits implantierten Schaft die gleiche Winkellage an einem gleich grossen Lagerkopf eingestellt werden. Eine solche Winkellage kann zusätzlich durch einen vom konischen Körper vorstehenden Stift mit einer dazu passenden Bohrung in der kugelförmigen Aufnahme des Lagerkopfes gesichert werden.

Eine weitere Möglichkeit für das Festsetzen des Kugelgelenks im Lagerkörper besteht in einem entgegengesetzt geschlitzten Kugelkörper mit konischer Bohrung, der auf einen zusätzlichen am konischen Körper angeformten Kreiskonus aufsteckbar ist und der durch einen Schlag auf den aufgesetzten Lagerkörper in diesem und selbsthemmend am konischen Körper festsetzbar ist.

Eine weitere Verbesserung der konischen Verbindung zwischen konischem Körper und der Gegenform im Schaft wird erreicht, wenn die Berührungspunkte in zwei zur Längsachse quer stehenden Ebenen verteilt sind und die beiden Ebenen einen vorgegebenen Mindestabstand zueinander aufweisen. Dies wird beispielsweise durch eine Unterbrechung der Eingriffsfläche im mittleren Bereich des konischen Körpers erreicht.

Im folgenden wird die Erfindung anhand von Ausführungsbeispielen beschrieben. Es zeigen:
- Fig. 1:: schematisch einen im Oberarmknochen implantierten Schaft;
- Fig. 2:: schematisch ein künstliches Schultergelenk mit einem Lagerkopf, der einen zum Schaft von Fig. 1 passenden konischen Körper aufweist;
- Fig. 3:: schematisch einen Querschnitt durch einen konischen Körper gemäss Fig. 2 mit einem elliptisch verlaufenden Umfang;
- Fig. 4:: schematisch einen Querschnitt durch einen konischen Körper gemäss Fig. 2, bei dem der Umfang einem Dreibogengleichdick entspricht;
- Fig. 5:: schematisch im Schnitt einen Lagerkörper, der durch einen Schlag über ein festsetzbares Kugelgelenk mit einem konischen Körper gemäss Fig. 2 verbinden lässt;
- Fig. 6:: schematisch eine Ansicht auf einen Kugelkörper und den konischen Körper von Fig. 5;
- Fig. 7:: schematisch im Schnitt einen Lagerkörper, bei dem ein Kugelgelenk zwischen Lagerkörper und einem konischen Körper mit einer in der Achsrichtung des konischen Körpers verlaufenden Schraube festsetzbar ist;
- Fig. 8:: schematisch einen in einem Oberarmknochen implantierten Schaft mit einer Gegenform für einen konischen Körper;
- Fig. 9:: schematisch einen zum Schaft von Fig. 8 passenden konischen Körper, der mit einer Kugelschale ein künstliches Schultergelenk zu einem Kugelkopf bildet, der über eine Plattform am Schulterknochen befestigt ist;
- Fig. 10:: schematisch einen Ausschnitt von Fig. 7 mit einem Druckkörper, der einen stiftförmigen Fortsatz für eine zusätzliche Sicherung im Lagerkörper aufweist;
- Fig. 11:: schematisch einen Probierlagerkopf für eine Anordnung gemäss Fig. 7;
- Fig. 12:: schematisch einen Schnitt gemäss Fig. 3, an dem eine drehfeste Verkeilung zwischen konischem Körper und Gegenform dargestellt ist;
- Fig. 13:: schematisch einen Schnitt durch einen konischen Körper mit einem ursprünglichen Rechteckquerschnitt, an dem nachträglich konische Teilflächen, welche im Schnitt elliptisch und passend zu einer Gegenform gemäss Fig. 12 hergestellt wuden; und
- Fig. 14:: schematisch einen konischen Körper gemäss Fig. 6, bei dem im mittleren Bereich der konischen Fläche eine Unterbrechung eingearbeitet ist.

In den nachfolgenden Ausführungsbeispielen sind gleiche Hinweiszeichen für gleiche Funktionen verwendet.

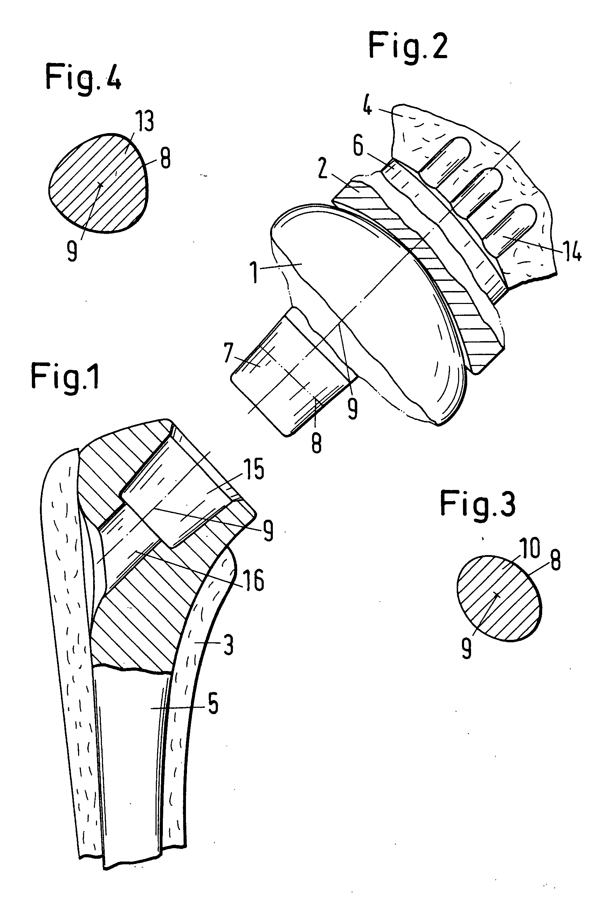

Ein erstes Ausführungsbeispiel ist in den Fig. 1 und 2 gezeigt. Ein Schaft 5 ist in einem Oberarmknochen 3 implantiert, wobei der Schaft 5 direkt in einem vorbereiteten Knochenbett verankert ist. Der Schaft kann aber ebensogut ein mit Knochenzement im Oberarmknochen verankerter Schaft sein. In Richtung einer Längsachse 9 für das eigentliche Schultergelenk ist eine Bohrung 16 angebracht, die in einer Gegenform 15 für einen konischen Körper 7 endet. Das eigentliche Gelenk wird durch einen mit dem konischen Körper 7 starr verbundenen Lagerkopf 1 und eine Lagerschale 2 gebildet, die ihrerseits mit einer im Schulterknochen 4 verankerten Plattform 6 starr verbunden ist. Zur Verankerung der Plattform 6 sind parallel zueinander Zapfen 14 an der Plattform 6 angebracht, die beispielsweise mit Knochenzement oder durch Presssitz in vorbereiteten Bohrungen des Schulterknochens 4 verankert sind.

Der konische Körper 7, und entsprechend die Gegenform 15, besitzt einen Querschnitt 10 mit Umfang 8, der gemäss Fig. 3 elliptisch ausgebildet ist. In Fig. 4 ist ein aus einem gleichseitigen Dreieck abgeleiteter Querschnitt gezeigt, der einem Dreibogengleichdick 13 entspricht, einer Form, die im Maschinenbau als Wellenverbindung verwendet wird.

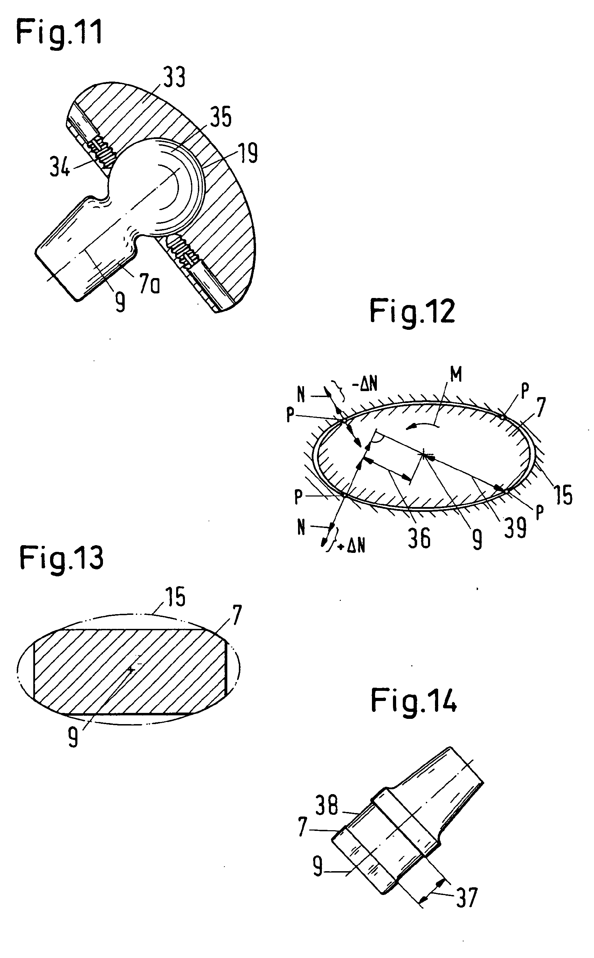

In Fig. 12 sind die angestrebten Verhältnisse bei einem annähernd elliptischen Querschnitt gezeigt. Es sind durch geringfügige Formabweichungen zwischen konischem Körper 7 und der Gegenform 15 vier Kontaktpunkte P vorgesehen, die sich bei intensiver Pressung zu Kontaktflächen erweitern. Ein radialer Abstand 39 eines Kontaktpunktes P ist so gewählt, dass eine Normalkraft N in einem Kontaktpunkt P mit ihrer Wirkungslinie in einem relativ grossen Abstand 36 an der Längsachse 9 vorbeiführt, um Anteile von einem Drehmoment M als Veränderungen von Normalkräften zu übertragen. So wird ein zusätzlich am konischen Körper 7 angreifendes Drehmoment M durch eine Abnahme der Vorspannung resp. durch eine Erhöhung der Vorspannung N um einen Bruchteil ΔN kompensiert. Der vorgespannte lokale Formschluss ist also entscheidend. In Fig. 13 ist die gleiche Situation extremer dargestellt. Der konische Körper 7 ist nur noch im Bereich der zu Kontaktflächen erweiterten Kontaktpunkte mit der elliptischen Grundform 15 in Berührung. Die restlichen Flächen sind zurückgesetzt.

Eine weitere Möglichkeit für die Abänderung des konischen Körpers 7 ist in Fig. 14 dargestellt. Um möglichst grosse Biegemomente in der Längsachse 9 übertragen zu können, findet die Verspannung in zwei Querschnitten statt, die um einen Mindestabstand 37 auseinanderliegen. Das heisst, der Konus hat im mittleren Bereich eine Unterbrechung 38 von diesem Mindestabstand 37.

Bei einem Dreibogengleichdick, wie es in Fig. 4 gezeigt ist, sind im Rahmen der Fertigungstoleranzen ebenfalls mehr als drei Kontaktpunkte angestrebt, wobei einige Kontaktpunkte mit den Wirkungslinien ihrer Normalkräfte ebenfalls in einem Abstand zur Längsachse 9 liegen sollten.

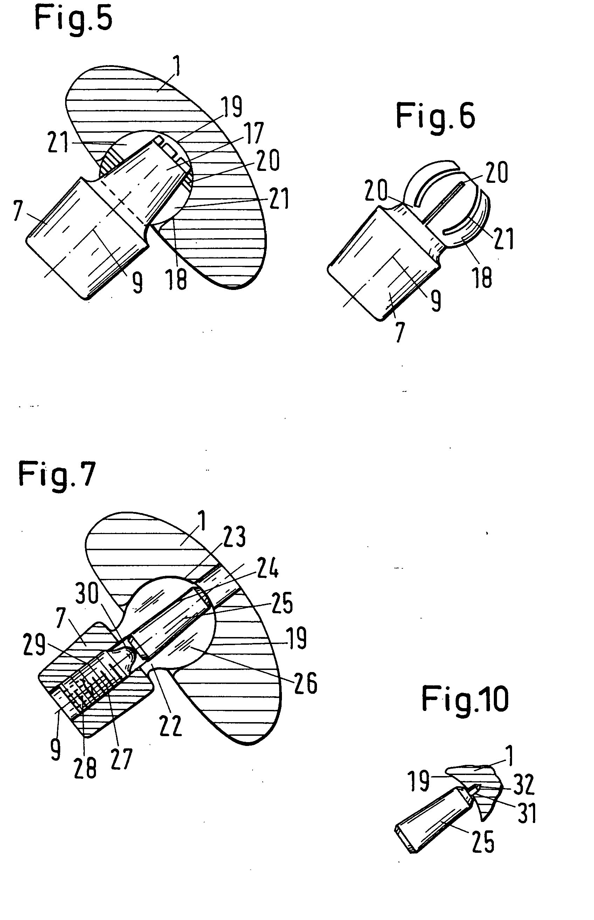

Im Beispiel der Fig. 5 und 6 ist die starre Verbindung zwischen konischem Körper 7 und Lagerkopf 1 durch ein festsetzbares Kugelgelenk realisiert. Der Lagerkopf besitzt eine kugelförmige Ausnehmung 19, die im Längsschnitt einen Winkel von mehr als 180° einschliesst. In diese Ausnehmung 19 ist ein komprimierbarer Kugelkörper 18 einsetzbar. Dieser ist komprimierbar, weil er eine durchgehende Innenbohrung und wechselseitig angebrachte Schlitze 21 aufweist, die an den Stirnseiten schmale Brücken 20 stehen lassen. Da die Innenbohrung als Kreiskonus ausgeführt ist, lässt sich der Kugelkörper 18 durch einen passenden Kreiskonus 17, der am konischen Körper 7 angeformt ist, aufspreizen. Dieser Kreiskonus 17 ist mit seinem Konuswinkel selbsthemmend gewählt, was zur Folge hat, dass bei einem Schlag auf den Lagerkopf 1 oder auf den konischen Körper 7 in Richtung der Längsachse 9 der Lagerkopf 1 gegenüber dem Kugelkörper 18 und der Kugelkörper 18 gegenüber dem konischen Körper 7 festgesetzt wird.

Ein weiteres Beispiel für ein festsetzbares Kugelgelenk zwischen Lagerkopf 1 und konischem Körper 7 ist in Fig. 7 gezeigt. An den konischen Körper 7 ist ein Hals 22 und eine Kugel 23 angeformt. Die Kugel 23 besitzt eine konische Bohrung 24 und Längsschlitze, die vom Scheitel bis in den Hals 22 gehen, sodass der Hals 22 in viele Biegeelemente aufgeteilt ist. Solange die konische Bohrung 24 leer ist, lässt sich die Kugel 23 zusammenpressen und in die kugelförmige Ausnehmung 19 des Lagerkopfes 1 einfahren. Erst wenn durch eine Gewindebohrung 29 im konischen Körper 7 ein konischer Druckkörper 25 eingeschoben und durch eine Schraube 27 vorwärts getrieben wird, können die durch Längsschlitze 26 getrennten Lamellen der Kugel 23 auseinandergespreizt werden, wobei die Halspartie eine Art Biegefeder und Fliessgelenk bildet. Statt der Schraube 27 kann auch ein Stössel eingeschoben werden, um mit einem Schlag auf den Stössel den konischen Druckkörper 25 festzusetzen. Die Schraube 27 ist als Madenschraube mit einem Innensechskant 28 versehen und treibt den konischen Druckkörper 25 mit einer Nase 30 vorwärts. Da wegen der kurzen Abmessungen im Halsbereich auch plastische Verformungen eintreten können, ist nur ein einmaliger Setzvorgang vorgesehen. Zu diesem Zweck werden gemäss Fig. 11 ein konischer Körper 7a und ein Probierlagerkopf 33, die äusserlich baugleich sind, im Schaft 5 (Fig. 1 und 2) eingesetzt, um eine optimale Stellung für den Probierlagerkopf 33 zu finden. Der konische Körper 7a besitzt einen starren Kugelkopf 35, an dem Madenschrauben 34 angreifen, um den Probierlagerkopf 33 in seiner optimalen Stellung zu fixieren. Anschliessend werden der konische Körper 7a und der Probierlagerkopf 33 vom Schaft 5 gelöst, um ausserhalb des Operationsfeldes die Lage vom Probierlagerkopf 33 zum konischen Körper 7a auf den endgültigen konischen Körper 7 und seinen Lagerkopf 1 (Fig. 7) zu kopieren. Wie so ein Kopiervorgang zur Erreichung einer gleichen relativen Lage aussehen kann, ist in der Patentanmeldung EP-A-0 931 522 gezeigt, wobei jedoch nur der vom Schaft lösbare konische Körper 7, 7a in der gleichen Vorrichtung eingespannt werden muss. Gemäss Fig. 10 kann in der optimalen Stellung eine Aussparung 32 in der kugelförmigen Ausnehmung 19 des Lagerkopfes angebracht sein, in welche ein stiftförmiger Fortsatz 31 des konischen Druckkörpers 25 als zusätzliche Drehsicherung hineinragt.

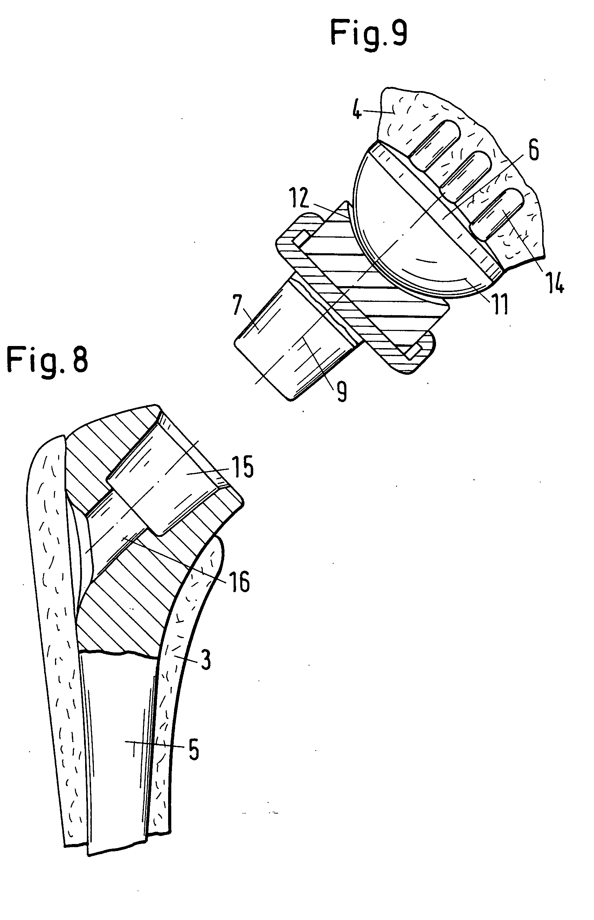

Im Beispiel von Fig. 8 und 9 sind die Funktionen von Kugel und Lagerschale vertauscht, um den Oberarm um einen Drehpunkt drehen zu lassen. Der im Oberarmknochen 3 implantierte Schaft 5 ist wiederum mit einer Bohrung 16 und mit einer Gegenform 15 für einen konischen Körper 7 versehen. Der konische Körper 7 ist jedoch verbreitert zu einer Aufnahme für eine Kugelschale 12, die ihrerseits einen Kugelkopf 11 teilweise umschliesst. Der Kugelkopf 11 ist durch eine Schnappverbindung oder Schraubverbindung (beide nicht gezeigt) auf einer Plattform 6 befestigt, die über Zapfen 14 im Schulterknochen 4 verankert ist. Die Verankerung der Plattform 6 kann ebenso über Knochenschrauben und vorstehende Rippen im Schulterknochen erfolgen.

In Fig. 14 ist ein konischer Körper 7 gezeigt, bei dem der tragende konische Teil eine Unterbrechung 38 aufweist. Auf diese Weise entstehen zwei konische Bereiche für die Verklemmung zu einer Gegenform 15 (nicht gezeigt), wobei diese Bereiche um einen Mindestabstand 37 auseinanderliegen, um Biegemomente in der Längsachse 9 übertragen zu können.

## Patentansprüche

1. Schultergelenkprothese mit zwei aufeinander gleitenden Lagerkörpern (1, 2; 11, 12) die zum Oberarm (3) mit einem Schaft (5) respektive zum Schulterknochen (4) mit einer Plattform (6) verbindbar sind, **dadurch gekennzeichnet, dass** die Verbindung zum Schaft (5) durch einen nicht rotationssymmetrischen, konischen Körper (7) mit einem selbsthemmenden Sitz erfolgt, wobei dessen Umfang (8) einen zu einer Längsachse (9) drehfesten und durch die Konizität verkeilten Formschluss mit einer Gegenform (15) im Schaft (5) bildet, damit die Verbindung lösbar und in gleicher Winkellage wiederholt festsetzbar ist.

2. Schultergelenkprothese nach Anspruch 1, **dadurch gekennzeichnet, dass** der Umfang (8) des konischen Körpers (7) und der Gegenform (15) elliptisch (10) ausgebildet ist.

3. Schultergelenkprothese nach Anspruch 1, **dadurch gekennzeichnet, dass** der Umfang (8) des konischen Körpers (7) und der Gegenform (15) als Dreibogengleichdick (13) ausgeführt ist.

4. Schultergelenkprothese nach einem der Ansprüche 1 bis 3, **dadurch gekennzeichnet, dass** an der Plattform (6) eine Lagerschale (2) und am Schaft (5) ein Lagerkopf (1) befestigt ist.

5. Schultergelenkprothese nach Anspruch 4, **dadurch gekennzeichnet, dass** der Lagerkopf (1) über ein festsetzbares Kugelgelenk mit dem konischen Körper (7) verbunden ist.

6. Schultergelenkprothese nach Anspruch 5, **dadurch gekennzeichnet, dass** an den konischen Körper (7) eine längsgeschlitzte (26) Kugel (23) mit einer konischen Bohrung (24) angeformt ist und dass der konische Körper (7) eine daran anschliessende Gewindebohrung (29) aufweist, um mit einer Schraube (27) und mit einem konischen Druckkörper (25) die Kugel (23) auseinander zu spreizen und in einer kugelförmigen Ausnehmung (19) des Lagerkopfes (1) festzusetzen.

7. Schultergelenkprothese nach Anspruch 6, **dadurch gekennzeichnet, dass** der konische Druckkörper (25) in seiner Endstellung mit einem stiftförmigen Fortsatz (31) als zusätzliche Drehsicherung in eine Aussparung (32) des Lagerkopfes (1) eingreift.

8. Schultergelenkprothese nach Anspruch 5, **dadurch gekennzeichnet, dass** der konische Körper (7) einen zusätzlichen Kreiskonus (17) aufweist, auf den ein aufspreizbarer Kugelkörper (18) selbsthemmend aufsteckbar ist, welcher in einer kugelförmigen Ausnehmung (19) des Lagerkopfes (1) durch Einschlagen vom Kreiskonus (17) verspannbar ist.

9. Schultergelenkprothese nach Anspruch 4, **dadurch gekennzeichnet, dass** die Plattform (6) mit verschieden grossen Lagerschalen (2) bestückbar ist.

10. Schultergelenkprothese nach einem der Ansprüche 1 bis 3, **dadurch gekennzeichnet, dass** an der Plattform (6) ein Kugelkopf (11) und am Schaft (5) eine Kugelschale (12) befestigt ist.

11. Schultergelenkprothese nach einem der Ansprüche 1 bis 10, **dadurch gekennzeichnet, dass** am konischen Körper (7) in Längsrichtung gesehen im mittleren Bereich die Kontur etwas zurückgenommen ist, um einen dieser Rücknahme entsprechenden Mindestabstand der Querschnitte zu erreichen, in denen eine Verspannung der konischen Flächen stattfindet.
